# EUROPEAN PATENT APPLICATION

(11) **EP 4 838 247 A1**
(43) Date of publication of application: **11.11.2026**
(21) Application number: 25175355.4
(22) Date of filing: 09.05.2025
(51) Int. Cl.: G01R 33/563, A61B 5/00

(54) **ACTUATOR FOR MAGNETIC RESONANCE ELASTOGRAPHY AND TISSUE OR ANIMAL SUPPORT DEVICE COMPRISING SUCH ACTUATOR**

(71) Applicant: Université Paris Cité, 75006 Paris (FR); Institut National de la Santé et de la Recherche Médicale, 75013 Paris (FR); Centre National de la Recherche Scientifique, 75016 Paris (FR); Assistance Publique - Hôpitaux de Paris, 75012 Paris (FR); Universität Heidelberg, 69117 Heidelberg (DE)
(72) Inventor: Sinkus, Ralph, 95620 Parmain (FR); Mangin, Gabrielle, 78300 Poissy (FR); Ducamp, Marguerite, 92800 Puteaux (FR); Boumaza, Asma, 75020 Paris (FR); Schregel, Katharina, 69221 Dossenheim (DE); Fels-Palesandro, Hannah, 69115 Heidelberg (DE); Zerelles, Roland, 69120 Heidelberg (DE)
(74) Representative: Demulsant, Xavier

(57) **Abstract**

A Magnetic Resonance Elastography device (1) for applying vibrations to a biological object such as an spheroids/organoids, tissue specimen and live animal like rodent which is positioned in the magnetic field of an MRI system with a transducer (7), the device comprising the transducer which is a rotating support plate (7A) for the biological object and a push-pull rod (10) to rotate the rotating support plate which transmits vibrations to the biological object, an actuator (6) consistent of a motor, two rotating bodies (13A, 14A) which support each a current-driven coil (13, 14) of conductive wire with a pair of terminals for electric connection to a current source, the rotating bodies having each an arm protruding from the body and mechanically linked to some transmission means (12) which are fastened to the push-pull rod, the rotating bodies being in opposite oscillatory motion by the application of an oscillatory current to the current-driven coils.

## Description

### FIELD OF THE INVENTION

The invention generally relates to Magnetic Resonance Elastography (MRE) and more particularly to pre-clinical MRE.

### BACKGROUND OF THE INVENTION

Elastography is an imaging technique which derives mechanical property maps from a visualization of shear waves propagating within soft biological tissues.

Magnetic Resonance Elastography (MRE), first described in 1995 and FDA-cleared 510(k) in 2009, has shown itself particularly useful, notably for studying the brain, or the detection and stage of liver fibrosis. In 2022 it was estimated that Magnetic Resonance Elastography (MRE) was deployed on more than 2000 MRI systems worldwide.

Several studies in humans reported physiological differences between brain areas, along with alterations in viscoelastic parameters in various diseases (e.g. tumour, hydrocephalus, neurodegenerative disease such as Alzheimer's disease, Multiple sclerosis, Parkinson's disease). Other studies explore applications for abdominal, pelvic, pulmonary, cardiac or musculoskeletal diseases, motivating preclinical application of Magnetic Resonance Elastography (MRE) in rodents, in vivo or ex vivo.

There is growing interest in the quantification of tissue biomechanics in health and disease. Magnetic Resonance Elastography (MRE) parameters render information on tissue microstructure not assessable with other imaging techniques.

Yet, the exact cellular and structural origin of the Magnetic Resonance Elastography (MRE) contrast is not fully understood.

For this, preclinical experiments in spheroids/organoids, tissue specimen and live rodents are crucial to elucidate the relation between biomechanical properties quantifiable with Magnetic Resonance Elastography (MRE) and histologically characterized tissue microstructure.

To date, there is no generic solution and no commercially available hardware for preclinical Magnetic Resonance Elastography (MRE). Instead, various custom-built in-house solutions are used by research groups across the world.

The exact designs of such custom-built hardware are not always shared, or are described incompletely in publications, diminishing reproducibility and generalization of results obtained with in-house hardware.

Such in-house solutions are generally dedicated to one single purpose, i.e., in vitro or in vivo application and in case of in vivo usually tailored to a specific organ.

For monochromatic wave propagation in the brain, the rodent head should be kept immobilized, with permanent contact with the actuator. Head locking is often secured with bite and/or ear bars (e.g. Atay et al. Measurement of the Dynamic Shear Modulus of Mouse Brain Tissue in Vivo by Magnetic Resonance Elastography, Journal of Biomechanical Engineering 130 (2): 021013*),* bite bars being generally integrated in the anaesthesia mask. Custom-made head-holders have also been developed, keeping the animals (young rats or mice) supine.

Magnetic Resonance Elastography (MRE) requires a mechanical actuator to generate shear waves within the biological tissue.

Several actuators for pre-clinical Magnetic Resonance Elastography (MRE) have been proposed to generate vibrations and induce waves in soft tissues of a small animal, or in a container of a soft biomaterial, within the bore of a pre-clinical scanner.

Actuators may be electromagnetic, pneumatic, or piezoelectric, as presented in 2018 by Bayly et al. Pre-clinical MR elastography: Principles, techniques, and applications, J Magn Reson Jun:291:73-83*.*

Electromagnetic actuator utilizes the main magnetic field B₀ in the scanner, an electromagnetic force being produced by placing a coil inside the MR bore. Applying alternating currents to an annular coil generates motion, a maximal torque acting on the coil if the normal vector of its plane is perpendicular to the B₀ filed, the resultant periodic tilt being transformed for mechanical actuation using a pivoted rod.

US5952828 (Mayo Foundation, 1999) disclose an electromagnetic transducer for MRE**,** comprising a coil of wire wound on a coil, the coil axis being perpendicular to the magnetic field B₀. The coil is mounted on a flexible arm and cantilevered from a supporting block. When a current passes through the coil, the magnetic field which is produced interacts with the field B₀, whose direction in mainly in the z-direction, hence it is strongly directional. The coil is thus twisted to bend the flexible arm upwards or downwards, depending on the direction of current flow. By alternating the direction of current flow, the coil twists back and forth to produce an alternating force which causes the flexible arm to oscillate. In one embodiment disclosed in this prior art document, the transducer can effect a dual transverse motion, and comprises two coils connected to a T-shaped drive shaft at pivot points, the central axis of each drive coil being oriented at right angle to B₀. Flexible connectors provide connection of the coils to a mounting block. When an alternating current is applied to the two coils, a transverse motion is obtained that is orthogonal to the magnetic field B₀. Importantly, in this description, whenever two coils are used, they operate in synchrony, i.e. their magnetic moments are aligned. Otherwise, the proposed constructs would not generate a shear motion.

An electromagnetic actuator allowing harmonic excitations with directions between 0° and 90° to the B₀ filed to be generated has been presented by Braun et al. Electromagnetic actuator for generating variably oriented shear waves in MR Elastography, Magnetic resonance in medicine 2003 Jul; Vol. 50 (1), pp. 220-2*.*

One clinical MRE electromagnetic actuator is presented by Feng et al. A multi-purpose electromagnetic actuator for magnetic resonance elastography, Magnetic Resonance Imaging, 51, 2018, 29-34*,* the actuator comprising a coil wired with its axial direction perpendicular to the B₀ field, the actuator comprising a vibration pad linked to the coil by a connection rod. By fixing the coil on a pivotable assembly, vibrations of the coil are transmitted to the pad by the connection bar.

Actuators proposed in prior art for MRE suffer from various disadvantages.

The compatibility of electromechanical actuators with MRI**,** due to the static field B₀ distortion they induce is problematic. The magnetic components of electromechanical or acoustic transducers compel their positioning outside the MRI bore. This relatively long distance (50-100 cm, depending on the fringe field) requires high transducer force and minimal friction in order to achieve efficient transmission.

Piezoelectric actuators lack amplitude, generate heat, and require high voltages which is often limiting when it comes to the amplification devices which are thus very expensive.

Phase lag in mechanical motion and difficulty in synchronization control are intrinsic problems of pneumatic actuators, as mentioned in document US11921183 (Sinkus, 2024).

Piezoelectric actuators, pneumatic drivers, or electrodynamic vibrations shakers have shortcomings: their actuation forces can be limited to either in vitro or in vivo use, safety considerations regarding the magnetic field need to be taken into account, and issues with mechanical transmission and/or upper harmonics may occur.

### SUMMARY OF THE INVENTION

The present invention is directed to addressing the effects of one or more of the problems set forth above. The following presents a simplified summary of the invention in order to provide a basic understanding of some aspects of the invention. This summary is not an exhaustive overview of the invention. It is not intended to identify key critical elements of the invention or to delineate the scope of the invention. Its sole purpose is to present some concepts in a simplified form as a prelude to the more detailed description that is discussed later.

It is an object of the invention to propose an actuator for Magnetic Resonance Elastography (MRE), more particularly for preclinic Magnetic Resonance Elastography (MRE), that allows generation of stable vibrations at high frequency range, typically from 100Hz to 1000Hz.

It is another object of the invention to propose an actuator for MRE, more particularly for preclinic MRE, that exhibit no parasite mechanical resonance in the frequency range from 100Hz to 1000Hz, thus reducing the risks of artefacts in data acquisition that may degrade the quality of the estimation of biomechanical properties of the tested tissue or organ.

It is another object of the invention to propose a support for MRE, more particularly a tissue or animal support for preclinic MRE, that can be used for various applications, by contrast to currently used in-house preclinical hardware, the support being able to be used for macroscopic applications such as rat brain, mouse brain, or micrometer applications such as organoids, or applications for thin tissue specimen, from millimeter to centimeter scale.

Another object of the invention is to propose a support for preclinic MRE, that is compatible with the existing equipment used for heating and monitoring of animals, as well as for inhalation anesthesia.

Another object of the invention is to provide a tissue or animal support for MRE that is fully MRI-compatible, without causing RF or other artefacts. The present invention relates on a Magnetic Resonance Elastography (MRE) device with a transducer for applying vibrations to a biological object such as an spheroids/organoids, tissue specimen and live animal like rodent, and which is positioned in the magnetic field of an MRI system,
the device comprising:
- the transducer comprising a rotating support plate which holds the biological object and which transmits vibrations to the biological object,
- a push- pull rod to rotate the rotating support plate,
- an actuator having a motor, two rotating bodies which support each a current-driven coil of conductive wire with a pair of terminals for electric connection to a current source,
- the rotating bodies having each an arm protruding from the body and mechanically linked to transmission means which are fastened to the push- pull rod, the rotating bodies being in oscillatory motion by the application of an oscillatory current to the current-driven coils,
- each rotating body with current-driven coil and arm oscillates around an axis in opposite directions of rotation relative to each other, between two limit angular positions, when a same sinusoidal current is applied by the current source, movement in which the magnetic momenta of the current-driven coils are orthogonal to the main magnetic field B₀ of the MRI system and in opposition to each other so that the total torque is zero,
- opposed oscillation of the rotating bodies which support the current-driven coils around their axis is transformed into translational oscillatory movement along a longitudinal axis of the push pull rod, by the mechanical connection between the arms and the transmission means.

Advantageously, he transmission means present flexible transmission means.

In some embodiments, the transmission means are made of polymer material, advantageously polyamide.

Advantageously, the transmission means presents at least one thin part with a restricted transversal section to be deformed elastically and which transmits the movement.

In some embodiments, the transmission means comprises a first part fastened to the push pull rod, and a second part comprising a T piece, at least the thin part with a restricted transversal section and two mounting fingers, each mounting finger being connected to one arm. Advantageously, the thin part between the second part and the mounting finger, has a material and a transversal section configured to be elastically deformed under the action of the arms on the mounting fingers allowing the back and forth translation movement of push pull rod.

Advantageously, the second part presents two thin parts.

In some embodiments, the transducer comprises a support plate movable around an axis with respect to a base plate of the device, the rotation axis of the support plate being transversal and perpendicular to the longitudinal movement of the push-pull rod along the longitudinal axis, the device comprising second rotative transmission means to transform the longitudinal movement of the push-pull rod into rotation of the support plate around its transversal axis.

Advantageously, the rotative second transmission means are flexible transmission means.

In some embodiments, the flexible transmission means presents a body with:
- at least one deformable part able to be elastically deformed,
- a main body which presents holes for rotating, fastened to the transducer,
- a connecting part fastened to the push pull rod and which is between the deformable part and the main body.

Advantageously, the deformable part elastically forms a thin region which presents a form which follows the radius of rotation around the axis.

In some embodiments, the push-pull rod is housed into a base plate which comprises on an upper part a bed/bench to receive a part of the animal, for instance the rear part, the front part of the animal being on the transducer.

Advantageously, the oscillatory current of the motor is at a frequency chosen among the frequency interval between 100Hz and 1000Hz.

In some embodiments, the transducer comprises a support plate, an interchangeable top unit being fastened to the support plate, the interchangeable top unit being chosen in the group comprising top unit for in vitro MRE or live rodents MRE.

Advantageously, the support plate presents several slides to position the interchangeable top unit.

In some embodiments, the interchangeable top unit presents:
- anesthesia cone for rodent, or
- a hole so that a piece, like a cylinder, which is an integral part of or attached to the support plate passes and transmits vibrations to the biological object.

In some embodiments, the interchangeable top unit presents a surface configured to receive mouse abdominal, rat brain mouse brain, thin specimen with centimeter scale or millimeter scale, organoid with micrometer scale.

While the invention is susceptible to various modification and alternative forms, specific embodiments thereof have been shown by way of example in the drawings. It should be understood, however, that the description herein of specific embodiments is not intended to limit the invention to the particular forms disclosed.

It may of course be appreciated that in the development of any such actual embodiments, implementation-specific decisions should be made to achieve the developer's specific goal, such as compliance with system-related and business-related constraints. It will be appreciated that such a development effort might be time consuming but may nevertheless be a routine understanding for those or ordinary skill in the art having the benefit of this disclosure.

### BRIEF DESCRIPTION OF THE DRAWINGS

The objects, advantages and other features of the present invention will become more apparent from the following disclosure and claims. The following non-restrictive description of preferred embodiments is given for the purpose of exemplification only with reference to the accompanying drawings in which:

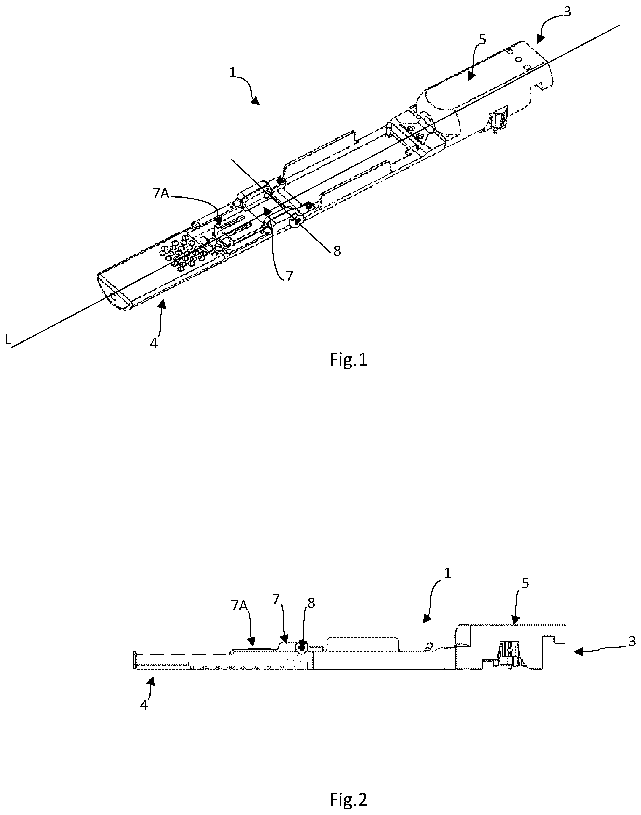

- Figure 1 is a perspective view of one embodiment of a Magnetic Resonance Elastography device comprising a long bed which can be used in conventional preclinical MRI scanners, a MR-compatible motor being visible on the right, and being connected to a transducer visible on the left via a push-pull rod;
- Figure 2 is a side view of the device represented in figure 1;
- Figure 3 is a perspective view of the device represented in figure 1, a rat-brain anaesthesia being placed on the device;
- Figure 4 is a longitudinal section and a transversal section of the device represented in figure 1;
- Figure 5 is a top view of the motor, comprising two current-driven coils which are oriented so that their corresponding magnetic momenta are oriented anti-parallel, the total torque on the motor being zero while both coils will oscillate in opposition;
- Figure 6 is a top view of an actuator prototype;
- Figure 7 is a detailed view of a flexible connection between the motor and the push-pull rod, this flexible connection allowing transformation of the rotative motion of both coils into a translatory motion of the push-pull rod in direction of B₀;
- Figure 8 is a perspective view of the flexing connection part between the motor and the push-pull rod;
- Figure 9 is a top view of the flexing connection between the motor and the push-pull rod;
- Figure 10 is a detailed view of a flexing connection, which allows a transformation of the translatory motion of the push-pull rod into a rotative motion of a support around its axis of rotation;
- Figure 11 is a perspective view of an elastic part of the flexing connection represented in figure 10;
- Figure 12 shows different applications of the device represented on figure 1, for macroscopic applications, such as rat-brain, mouse-brain, or abdominal experiments, or for experiments on organoids (micrometer scale) and thin tissue specimen (millimeter or centimeter scale), the front-end of the device being easily changed and adapted to different needs and imaging scenarios;
- Figure 13 is a graph representing the variation of acceleration g with time, measured by an accelerometer fixed to the transducer, for a frequency of 100 Hz (top) of the oscillatory current of the motor, and the Fourier transform of the data from the first graph (bottom);
- Figure 14 to 22 are graphs similar to the ones of figure 13, for a frequency of 200, 300, 400, 500, 600, 700, 800, 900 and 1000 Hz.

### DETAILED DESCRIPTION OF ILLUSTRATIVE EMBODIMENTS

The device 1 represented on the figures is advantageously able to be connected on existing fixation device, typically used for securing cradles, and can thereby be moved into the MRI.

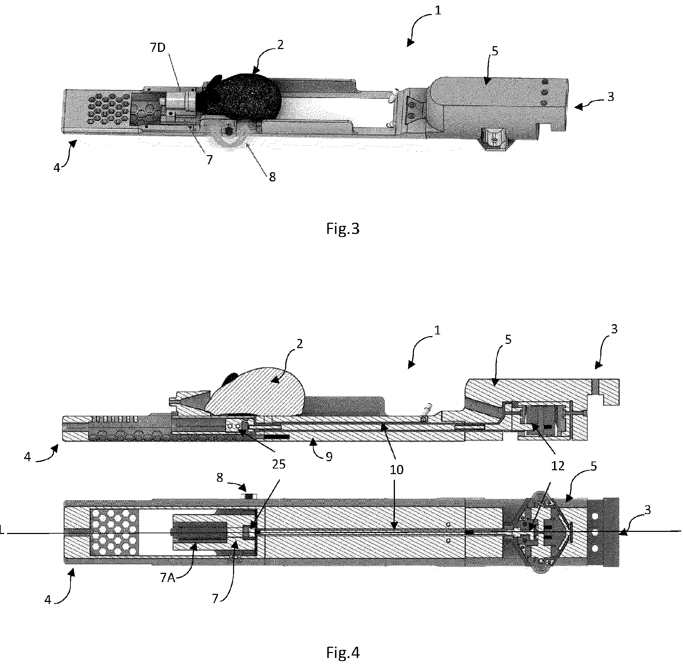

On figure 3 and 4, an animal 2, such as a mouse, for example for acquisition of brain MRE, is resting on the device 1.

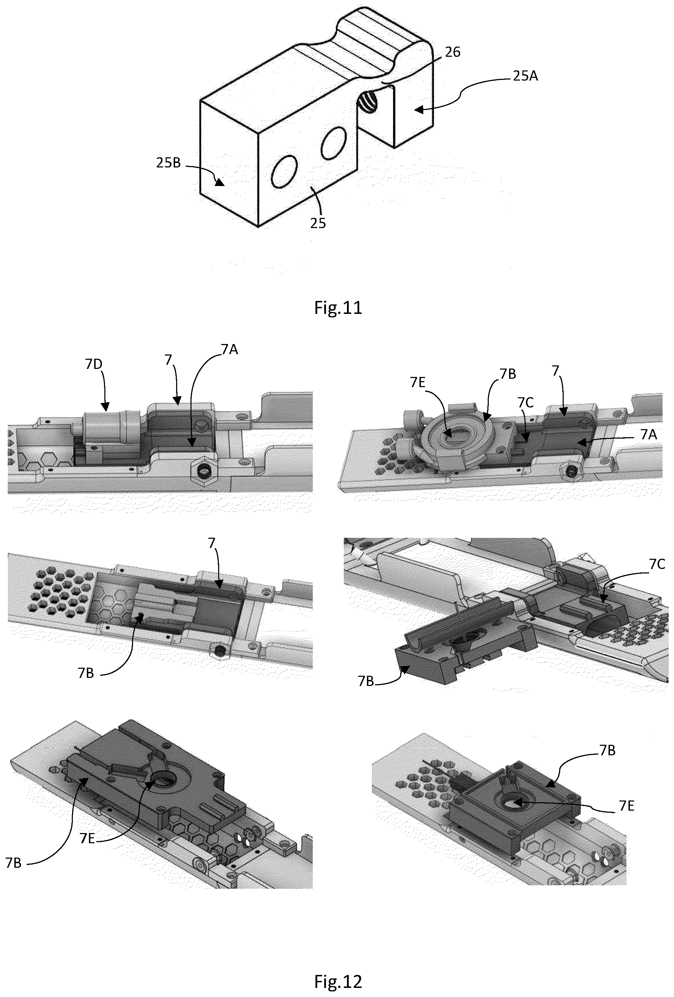

As represented on figure 12, various front-end components can be fixed on the device 1, for various biological objects and applications.

The biological objects can be chosen for instance among: rat-brain, mouse-brain, mouse-abdominal, organoid, or thin specimen on the millimeter scale or on the centimeter scale.

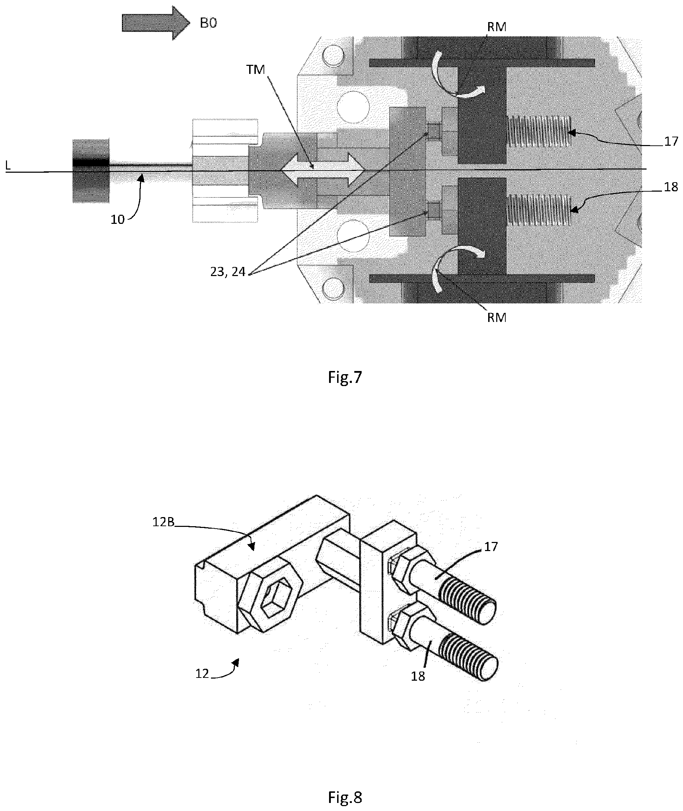

The device uses a motor which is based on the same electromagnetic concept of current driven coils within the main B₀ field of the MRI as described beforehand. However, the main difference compared to prior art is that here the two coils operate in opposition, which eliminates all transverse motion and leaves only longitudinal motion (see TM in figure 7) to generate a forward-backward motion of the push-pull rod 10. The consequence is that residual EM stray fields on the axis of symmetry, which is the axis were MRI scanning is performed, are zeroed out, minimizing any stray field effects on the imaging.

The idea of using two coils in opposition has the additional advantage of a net zero torque of the motor block. Thus, even when operating under a sinusoidal current, the motor block does not rotate as a whole and therefore rests mechanically stable without "rattling". All what is generated is a linear forward-backward motion. Also, because the torques are additive in the forward-backward direction, the force is doubled leading to sufficient strength to generate vibrations of sufficient amplitude even under load. In MRE it is essential to generate stable vibrations of sufficient amplitude. This is not simple in the context of preclinical Magnetic Resonance Elastography (MRE) as vibrations need to be at a high frequency range (100-1000 Hz), which realizes the multi-purpose device of the invention.

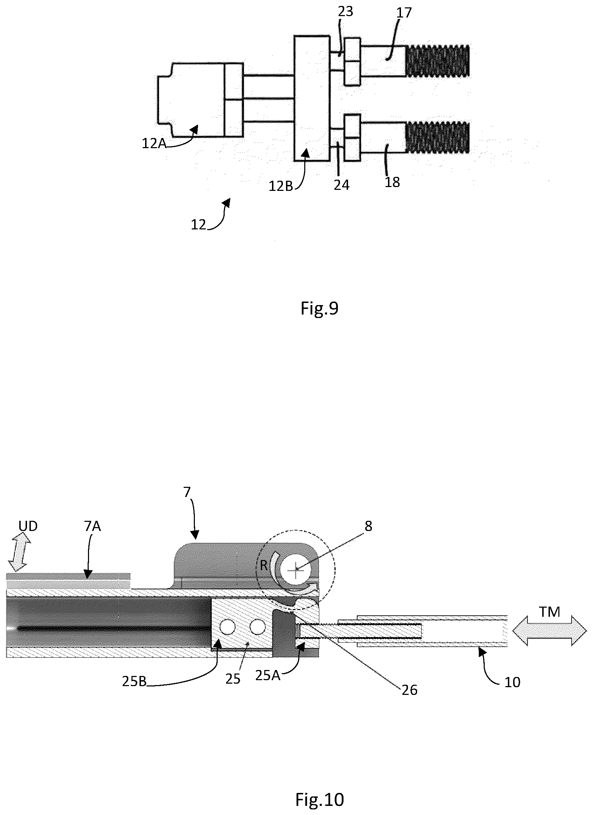

The animal (here the example for acquisition of brain MRE) is resting on the transducer, which moves around an axis of rotation to induce mechanical vibrations in the brain, i.e., the transducer will perform an Up-Down motion around the axis of rotation (see UD on figure 10) and thereby "shake" the mouse head.

This induces mechanical waves in the animal's brain. This motion is essential for performing elastography.

The rotation is generated via an electromagnetic (EM)-motor located sufficiently far away from the region of interest to avoid any image perturbations from residual stray fields. Motor and transducer are connected to each other via a push-pull rod and flexible joints.

The invention describes details of the EM-motor as well as in the details of the flexible joints, i.e., how the motion of the moving elements within the motor is transmitted to the transducer.

The device 1 has two extreme longitudinal parts 3, 4.

The first extreme longitudinal part 3 comprises a casing 5 for an actuator 6, the second longitudinal part 4 comprising a rotating support plate 7A for the fixation of front-end components, the support plate 7A having a transverse axis of rotation 8 with respect to a base plate 9 and bed/bench of the device 1 inside which passes a push-pull rod 10.

The push-pull rod 10 is moveable in translation along a longitudinal axis L of the device 1, the longitudinal translation movement of the push-pull rod 10 (see TM on figure 7 and 10) being transformed to a rotation of the rotating support plate 7A around the axis 8 in a first sense of rotation (due to the push) and a second sense of the rotation (due to the pull) opposed to the first sense of rotation, and so on (see UD on figure 10).

The actuator 6 comprises an electromagnetic motor 11 and a flexible transmission 12 connecting the motor 11 to the push-pull rod 10.

The electromagnetic motor 11 is located sufficiently far away from the region of interest, thanks to the great length of the push-pull rod 10 (for example several tens of centimeters), to avoid any image perturbations from residual stray fields. Furthermore, due to the concept of two coils in opposition, the residual EM stray field of motor is minimized on the z-axis due to symmetry. Mind that advantageously the angular motion of the two coils is minimal (just a few degrees) having hence the two magnetic moments of each coil almost always in exact opposition.

The electromagnetic motor 11 comprises two rotating bodies 13A, 14A with current-driven coils 13, 14.

A same current arrives and is divided into two in the two driven current coils 13, 14, and the sub-current of the current-driven coil 13 is in opposition in regard to the sub-current in the other coil 14.

Advantageously, the two rotating bodies 13A, 14A with coils 13, 14 are of identical structure (same rotating body, same number and thickness of coils 13 and 14) and the device 1 is placed in the MRI during MRE such that the coils 13, 14 are oriented within the main magnetic field B₀ of the MRI, the corresponding magnetic momenta M1, M2 of the coils 13, 14 being thus in opposition to each other and orthogonal to B₀ and cancel each other so that the total torque generated by B₀ on the two coils 13,14 is zero.

Each rotating body 13A, 14A with individual coil 13, 14, when a sinusoidal current is applied, oscillates around its corresponding axis 15, 16 between two limit angular positions of the rotation.

For the first sense of rotation of the transducer 7, the rotating body 13A rotated in a sense contrary to the sense of the rotating body 14A.

Then in the second sense of rotation of the transducer 7, it is the same thing but each rotating body 13A, 14A change their sense of rotation relative to their sense of rotation they have for the first sense of rotation of the transducer 7.

This specific configuration leads to a total torque of the electromagnetic motor 11 which is zero.

Thereby, when operating within the main magnetic field B₀ of the MRI system, the electromagnetic motor 11 will not try to turn or rotate around its own axis at all. Hence, the electromagnetic motor 11 represents a very stable mechanical part.

The flexible transmission 12 serves to translate the rotative motion of the two rotating bodies 13A, 14A with oscillating coils 13, 14 and a mechanical connecting arm 19, 20 into a translatory motion which is moving the push-pull rod 10.

The transmission means 12 comprises a first part 12A fastened to the push- pull rod 10, and a second part 12B with a T piece and at least one thin part 23, 24 with a restricted transversal section and two mounting fingers 17, 18, each mounting finger 17, 18 being connected to one arm 19, 20 protruding from one body with coil 13, 14.

One mounting finger 17 is passing through a hole of the arm 19, the other mounting finger 18 is similarly passing through a hole of an arm 20 of the other body with coil 14.

In the represented embodiment, each mounting finger 17, 18 has a thread for mounting a nut 21, 22. Other fixation means, such as gluing, welding, may be considered.

Advantageously, both rotating bodies with coils 13, 14 operate and rotate around their axis in synchronicity, in opposite direction, by running the coils 13, 14 in parallel electrically, with respect to a same applied sinusoidal current.

Thus, when the device 1 is placed in an MRE device, the axis of the coils 13, 14 being perpendicular to B₀ of the MRI, there is a coupling between the magnetic field B₀ of the MRI and the magnetic field b₁₃ and b₁₄ created by the passage of the sinusoidal current in the coil 13,14 which generates a force which causes the movement of the rotating bodies 13A and 14A. The fact that it is a sinusoidal current allows to obtain a synchronous rotation of the coils 13, 14 around their axis 15, 16.

Then this rotation movement is transformed to a longitudinal displacement of the push-pull rod 10 by the flexible transmission 12 along the longitudinal axis L.

Without B₀ of the MRI, there is no movement of the rotating bodies 13A, 14A and no vibrations generated with the push-pull rod 10, even if there is the passage of the sinusoidal current in the coils 13, 14.

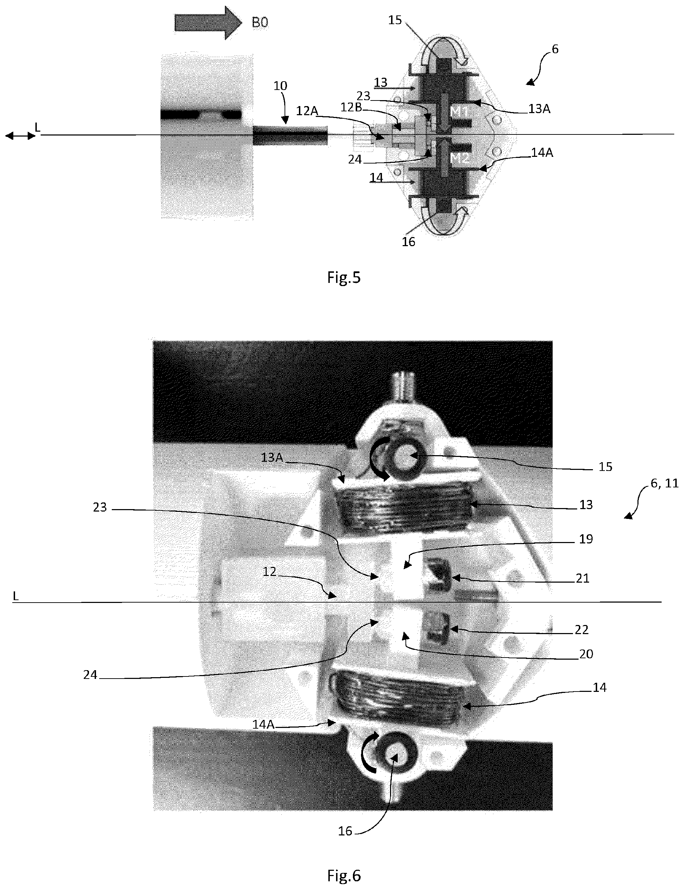

In the represented embodiment, for instance on figure 6, elastic deformation of the flexible transmission 12 is localized, thanks to the presence of thinned sections 23, 24 in the material forming the flexible transmission, e.g. a polymer material such as polyamide.

The transversal section of the thin part 23, 24 is configured to resist to the fatigue but to be deform elastically to transmit the movement of the arm 19, 20, and the mounted fingers 17,18 to the push-pull rod 10.

For instance, in regard to the longitudinal axis L, the transversal section of the thin part 23, 24 can be at least three times smaller than the transversal section of the horizontal branch of the T piece and/or the transversal section of the arm 19,20.

The second part 12B with a T piece can be slightly deformed elastically too, as represented on figure 6, but less elastically deformed than the thin part 23, 24.

As represented on figure 10, this translatory motion TM of the push-pull rod 10 is transformed into a rotative movement of the rotating support plate 7A around the axis 8, thanks to second flexible transmission means 25.

More precisely, when the push-pull rod 10 moves forth, the rotating support plate 7A moves in rotation upwardly, and when the push-pull rod 10 moves back, the rotating support plate 7A moves in rotation downwardly (see UD on figure 10).

The second flexible transmission means 25 presents a body with:
- at least one deformable part 26 able to be elastically deformed,
- a main body 25A which presents holes for rotating,
- a connecting part 25B fastened to the push- pull rod 10, and which is between the deformable part 26 and the main body 25A.

The connecting part 25B presents a thread to receive the push- pull rod 10.

Advantageously, to minimize strain in the second flexible transmission 25, the deformable part 26 elastically forms a thin region which presents a form which follows the radius of (~17mm radius) around the axis 8.

The transversal section of the deformable part 26 is configured to resist to the fatigue but to be deform elastically to transmit the movement of the push pull rod 10 and the connecting part 25B to the main body 25A fastened to the transducer 7.

For instance, in regard to the longitudinal axis L, the transversal section of the deformable part 26 can be at least three times smaller than the transversal section of the connecting part 25B and/or the transversal section of the main body 25A.

As represented in figure 12, the device 1 can be used for different applications and can easily be served via interchangeable top units 7B, e.g. fixed on the support plate 7A via a plastic screw.

Each unit is tailored to a specific purpose enabling to acquire MRE in vitro (tissue specimen, spheroids, organoids, Cancer-on-Chip) and in live rodents (mouse and rat; various organs).

The interchangeable unit 7B can be a bed composed of a frame with a bench for animal positioning. The frame is compatible with standard equipment used for heating and monitoring of animals as well as inhalation anesthesia.

The transducer 7 can be equipped with two guiding or sliding rails 7C, which ensure the exact and reproducible positioning of the top unit. The top unit is then fixated via a plastic screw.

The exchange of top units 7B takes less than a minute.

Moreover, the interchangeable top unit 7B can present:
- anesthesia cone 7D for rodent, or
- a hole 7E so that a piece, like a cylinder, which is an integral part of or attached to the support plate 7A passes through the hole 7E and transmits vibrations to the biological object which is on upper part of the interchangeable top unit 7B.

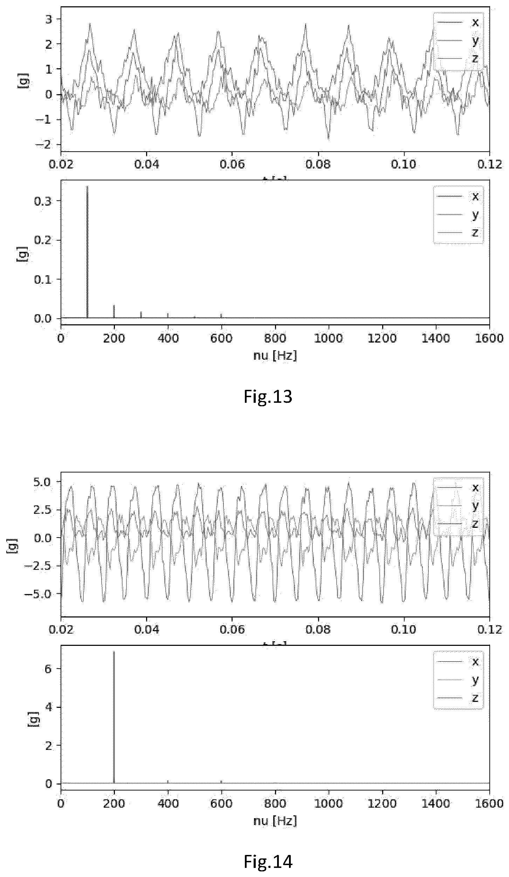

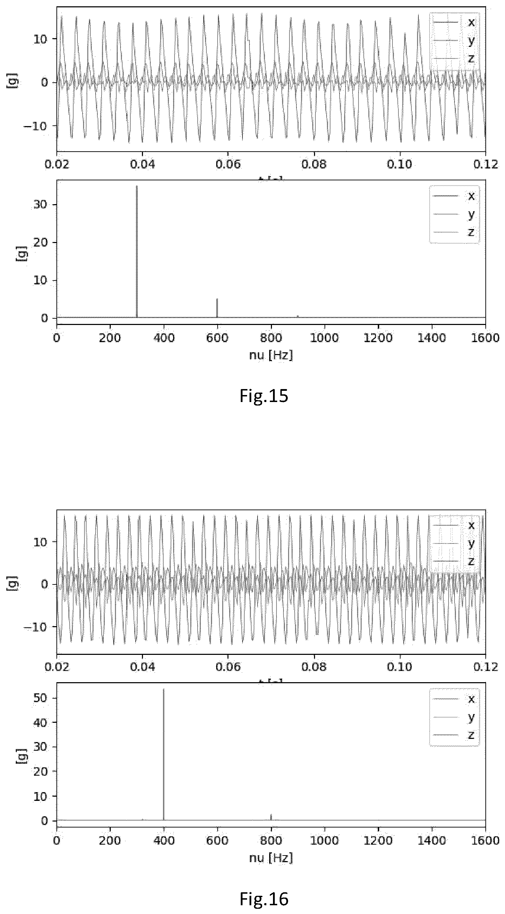

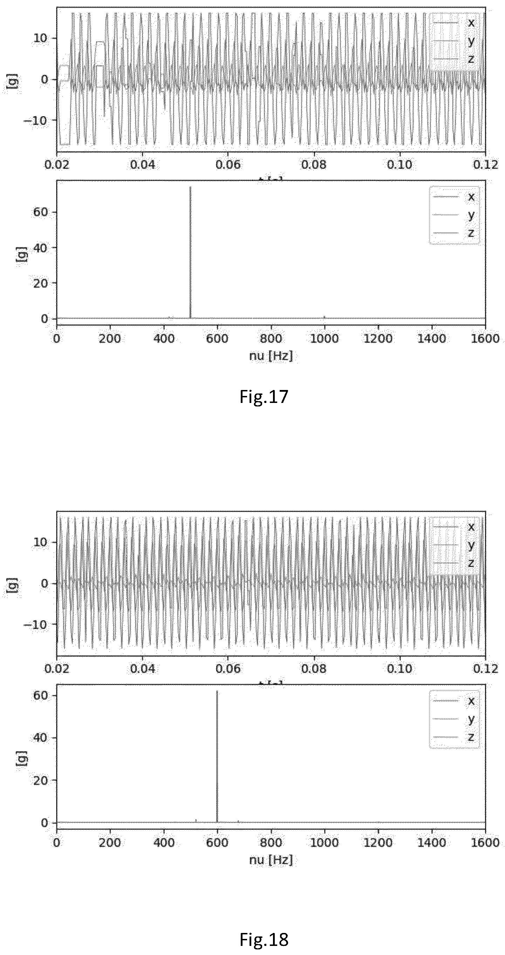

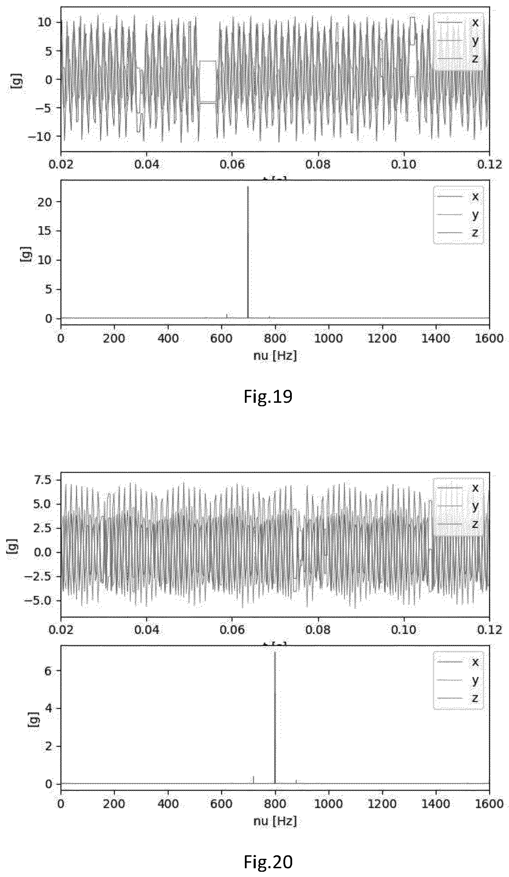

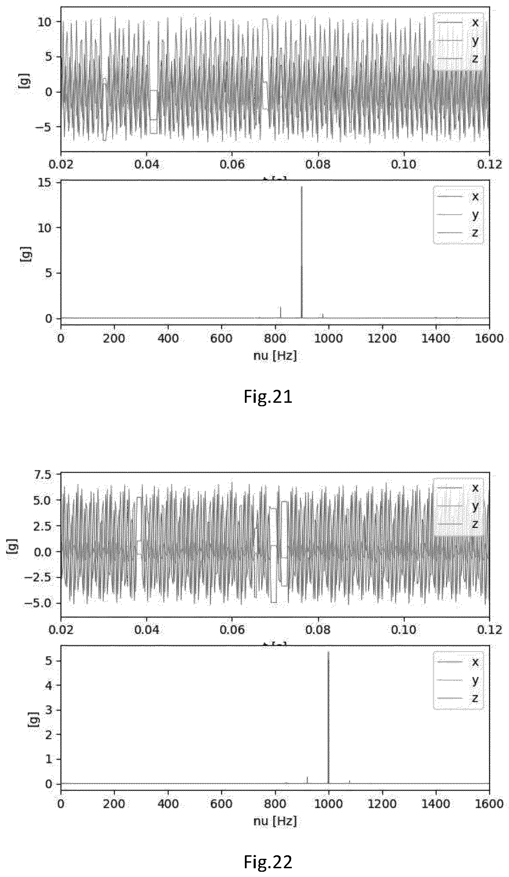

As demonstrated in figures 13-22, the device exhibits minimal mechanical resonance frequencies in the range from 100Hz-1000Hz.

On each figure 13 to 22, the Fourier transform of the mechanical vibrations essentially shows that only one frequency is produced on the transducer, and this frequency is the same that the frequency which is imposed by the oscillatory current of the motor.

The present invention has many advantages.

The electromagnetic motor, advantageously comprising two coils operating in opposition have zero net torque and is fully MR-compatible, without causing RF or other artifacts.

A first flexing connection allows to translate the rotative motion of the two coils into a translatory motion.

A second flexing connection allows to translate the translatory motion of the push-pull rod into a rotative motion by minimizing strain on the flexing parts.

These two flexing connections allow to transmit without any play and hence without any loss, the motion towards the push-pull rod.

In contrast to currently used in-house preclinical MRE hardware, a versatile design of the front end enables different applications ranging from in-vivo to ex-vivo and from microscale to macroscale.

The bed of the device is similar to the standard bed distributed by the Bruker company, and thus compatible with and usable in most Bruker MRI scanners including those equipped with an AutoPacTM^{®} system.

The frame is compatible with standard equipment used for heating and monitoring of animals as well as inhalation anesthesia.

The support 1 may be produced using 3D-printed, at low cost, for preclinical MR-Elastography, featuring plug-and-play top units that enable the quantification of biomechanics in spheroids, organoids, Cancer-on-Chip, ex vivo tissue specimen, and various organs in rodents.

Moreover, the device of the present invention increases generalizability of results across scales (in vitro to in vivo) and reproducibility as mechanical shear waves crucial for MRE are elicited in the same robust way in all applications. A generic solution is provided for multiple purposes: in vitro and in vivo MRE is possible with one single set-up via interchangeable top units.

The device exhibits no mechanical resonance frequencies in the range from 100Hz-1000Hz as they would lead to artifacts in the data acquisition and thus degrade the quality of the estimation of the biomechanical properties. The present invention provides a robust and stable method to generate stable vibrations of sufficient amplitude at high frequencies (for instance 100-1000Hz) at a given frequency to elicit mechanical shear waves crucial for MRE.

The device, in particular the coils, does not warm up and it is secured for the IRM.

## Claims

1. A Magnetic Resonance Elastography device (1) with a transducer for applying vibrations to a biological object such as an spheroids/organoids, tissue specimen and live animal like rodent, and which is positioned in the magnetic field of an MRI system, wherein the device (1) comprising:
- the transducer (7) comprising a rotating support plate (7A) which holds the biological object and which transmits vibrations to the biological object,
- a push- pull rod (10) to rotate the rotating support plate (7A),
- an actuator (6) having a motor, two rotating bodies (13A, 14A) which support each a current-driven coil (13, 14) of conductive wire with a pair of terminals for electric connection to a current source,
- the rotating bodies (13A, 14A) having each an arm (19, 20) protruding from the body and mechanically linked to transmission means (12) which are fastened to the push- pull rod (10), the rotating bodies (13A, 14A) being in oscillatory motion by the application of an oscillatory current to the current-driven coils (13, 14),
- each rotating body (13A, 14A) with current-driven coil (13, 14) and arm (19, 20) oscillates around an axis (15, 16) in opposite directions of rotation relative to each other, between two limit angular positions, when a same sinusoidal current is applied by the current source, movement in which the magnetic momenta of the current-driven coils (13, 14) are orthogonal to the main magnetic field B₀ of the MRI system and in opposition to each other so that the total torque is zero,
- opposed oscillation of the rotating bodies (13A, 14A) which support the current-driven coils (13, 14) around their axis (15, 16) is transformed into translational oscillatory movement along a longitudinal axis (L) of the push pull rod (10), by the mechanical connection between the arms (19, 20) and the transmission means (12).

2. A Magnetic Resonance Elastography device (1) according to claim 1, wherein the transmission means present flexible transmission means (12).

3. A Magnetic Resonance Elastography device (1) according to claim 2, wherein the transmission means (12) are made of polymer material, advantageously polyamide.

4. A Magnetic Resonance Elastography device (1) according to claim 2 or 3, wherein the transmission means (12) presents at least one thin part (23, 24) with a restricted transversal section to be deformed elastically and which transmits the movement.

5. A Magnetic Resonance Elastography device (1) according to claim 4, wherein the transmission means (12) comprises a first part (12A) fastened to the push pull rod (10), and a second part (12B) comprising a T piece, at least the thin part (23, 24) with a restricted transversal section and two mounting fingers (17, 18), each mounting finger (17,18) being connected to one arm (19, 20).

6. A Magnetic Resonance Elastography device (1) according to claim 5, wherein the thin part (23, 24) between the second part (12B) and the mounting finger (17, 18), has a material and a transversal section configured to be elastically deformed under the action of the arms (19, 20) on the mounting fingers (17, 18) allowing the back and forth translation movement of push pull rod (10).

7. A Magnetic Resonance Elastography device (1) according to any of claims 5 or 6, wherein the second part (12B) presents two thin parts (23, 24).

8. A Magnetic Resonance Elastography device (1) according to any of claims 1 to 7, wherein the transducer (7) comprises a support plate (7A) movable around an axis (8) with respect to a base plate (9) of the device (1), the rotation axis (8) of the support plate (7A) being transversal and perpendicular to the longitudinal movement of the push-pull rod (10) along the longitudinal axis (L), the device (1) comprising second rotative transmission means to transform the longitudinal movement of the push-pull rod (10) into rotation of the support plate (7A) around its transversal axis (8).

9. A Magnetic Resonance Elastography device (1) according to claim 8, wherein the rotative second transmission means are flexible transmission means (25).

10. A Magnetic Resonance Elastography device (1) according to claim 9, wherein the flexible transmission means (25) presents a body with:
- at least one deformable part (26) able to be elastically deformed,
- a main body (25A) which presents holes for rotating, fastened to the transducer (7),
- a connecting part (25B) fastened to the push pull rod (10) and which is between the deformable part (26) and the main body (25A).

11. A Magnetic Resonance Elastography device (1) according to claim 10, wherein the deformable part (26) elastically forms a thin region which presents a form which follows the radius of rotation around the axis (8).

12. A Magnetic Resonance Elastography device (1) according to any one of claim 1 to 11, wherein the push-pull rod (10) is housed into a base plate (9) which comprises on an upper part a bed/bench to receive a part of the animal, for instance the rear part, the front part of the animal being on the transducer (7).

13. A Magnetic Resonance Elastography device (1) according to any one of claims 1 to 12, wherein the oscillatory current of the motor is at a frequency chosen among the frequency interval between 100Hz and 1000Hz.

14. A Magnetic Resonance Elastography device (1) according to any one of preceding claims, wherein the transducer (7) comprises a support plate (7A), an interchangeable top unit (7B) being fastened to the support plate (7A), the interchangeable top unit (7B) being chosen in the group comprising top unit for in vitro MRE or live rodents MRE.

15. A Magnetic Resonance Elastography device (1) according to claim 14, wherein the support plate (7A) presents several slides (7C) to position the interchangeable top unit (7B).

16. A Magnetic Resonance Elastography device (1) according to claims 14 or 15, wherein the interchangeable top unit (7B) presents:
- anesthesia cone (7D) for rodent, or
- a hole (7E) so that a piece, like a cylinder, which is an integral part of or attached to the support plate (7A) passes and transmits vibrations to the biological object.

17. A Magnetic Resonance Elastography device (1) according to any one of claims 14 to 16, wherein the interchangeable top unit (7B) presents a surface configured to receive mouse abdominal, rat brain mouse brain, thin specimen with centimeter scale or millimeter scale, organoid with micrometer scale.
